# EUROPEAN PATENT APPLICATION

(11) **EP 2 272 458 A1**
(43) Date of publication of application: **12.01.2011**
(21) Application number: 09008946.7
(22) Date of filing: 09.07.2009
(51) Int. Cl.: A61C 5/00

(54) **Dental product comprising at least one veneer**

(71) Applicant: Nobel Biocare Services AG, 8058 Zürich-Flughafen (CH)
(72) Inventor: Karlsson, Per-Olof, 44165 Alingsås (SE); Nilsson, Urban, 44295 Hålta (SE); Melin, Daniel, 41649 Göteborg (SE)
(74) Representative: Byström, Kurt Linus

(57) **Abstract**

The invention concerns an individualized dental product (2) ready for application comprising at least one tooth veneer (2) which is of a ceramic material, is plate-like and has a plate thickness of at least about 0.08 mm, wherein the thickness is less than 0.2 mm, less than 0.14 mm or less than 0.1 mm. The invention further concerns a method of improving the aesthetics of a tooth (3), wherein the method comprises placing a dental product (2) onto a tooth surface.

## Description

### FIELD OF THE INVENTION

The present invention relates to a dental product comprising at least one veneer. In dentistry, a veneer is a thin layer of restorative material placed over a tooth surface, to improve the aesthetics of the tooth. Consequently, the invention is also related to a method for improving the aesthetics of a tooth.

### BACKGROUND OF THE INVENTION

US 2007/0298381 A1 describes a dental product for teeth coloring and teeth coverage. The dental product includes a solid tooth veneer which has cervical, proximal and incisal edges. This dental veneer is used for temporary attachment to a tooth which has had other restorative dentistry, such as permanent veneers or crowns. The veneer is placed on the tooth having an undesirable appearance in order to alter the appearance of the tooth by displaying a different color, size or shape than the underlying tooth. The tooth veneer is made from a polymer material which is non-toxic and non-water soluble, and preferably from an ethyl cyanoacetate based polymer. The temporary veneer remains on the tooth of the user for preferably up to seven days, but may be used for less than one day or even less than one hour. The veneer is attached by a non-permanent adhesive type material and can be removed by simply pulling the tooth cover off the individual tooth. Apart from the earlier carried out restorative dentistry, the tooth is not physically or chemically altered by applying and removing this polymer tooth veneer.

US 2009/0004629 A1 describes a tooth veneer in the form of a dental arch laminate which can be made of plastic, such as durable, injection-molded plastic, or a veneer form of plastic. These dental arch laminates are configured for comfortable and reusable application to a person's teeth. The arch laminates are fitted and adhered using a non-toxic tackification adhesive which is not a bonding agent, cement or other semi-permanent adhesive. Rather, the non-toxic tackification adhesive is temporary and reusable, such that a person can easily reapply the non-toxic tackification adhesive, for instance, for daily use.

Each of the two known veneers discussed above are made of a polymer. This allows the veneer to have a thickness from approximately 0.1 mm and more, up to 0.9 mm.

US 2005/0227204 A1 describes a reusable veneer for temporary use which can be removed by the use of a warm water rinse in the mouth and pulling the veneer off from the teeth. These temporary veneers can be made of porcelain, plastic, other semi-rigid composite materials, or combinations thereof. These veneers can be made from 0.2 mm to about 1 mm in thickness.

None of the above-described veneers require preparation and pre-shaping of a tooth, including cutting, drilling, grinding and other forms of permanently removing material from a tooth. It is understood that these veneers can thus be applied to non-prepared teeth. This is generally regarded as advantageous as irreversible alterations are often a traumatic experience for a person and may require pain-killing shots to be used prior to the procedure. This would also increase costs of the application of a veneer.

Clearly, a non-invasive or minimally invasive application of a veneer has a huge psychological advantage over application of a veneer which requires permanent removal of tooth material.

Although the temporary veneers are certainly very useful, many people still find the need to apply, remove and re-apply veneers inconvenient, time-consuming, and they dislike the risk of forgetting to apply and/or to have at hand the necessary materials to apply the veneer. Also, temporary veneers provide relatively poor aesthetics. Hence, there is a need for providing a permanent veneer which does not require any traumatic invasive preparation of the selected tooth.

### SUMMARY OF THE INVENTION

The present invention provides an individualized dental product comprising at least one tooth veneer, which is of a ceramic material, is plate-like and has a plate thickness of at least about 0.08 mm, wherein the thickness is less than 0.2 mm, less than 0.14 mm or even less than 0.1 mm. The phrase "individualized" is understood to mean that the dental product has characteristics which are related to the characteristics of a tooth of an individual. For a veneer this often implies that one side thereof is designed to match a surface of the tooth it is meant to cover. Given the match, there will not be a need to remove material from the tooth. Given the thickness of the plate of the tooth veneer, there is also no need to remove material from the tooth. Hence, the veneer may be referred to as a "non-preparatory" veneer. Placement of the veneer onto the selected tooth does not uncomfortably affect spatial arrangements within the set of teeth and the mouth of the respective person. Making the tooth veneer of a ceramic material enables permanent use of the tooth veneer as the tooth material can be made to sustain the environment in the mouth of a person throughout his/her lifetime.

Despite a tailor-made production of the tooth veneer, the handling and the correct application, i.e. fixing of the tooth veneer to the tooth, may require the skills of a dental practitioner.

In an embodiment of a dental product according to the invention, each of the at least one tooth veneer is designed to match a part of a predetermined tooth that is to be covered by the veneer. Advantageously, the actual appearance of the shape of the tooth covered by the veneer, will consequently not differ, as compared with the shape of the tooth before covering with the veneer. The veneer merely provides improvement in the appearance of the color of that tooth.

In an embodiment of a dental product according to the invention, each of the at least one veneer is individually designed to correspond to a part of a non-prepared tooth that is to be covered by that veneer. Advantageously, great care can be taken in ensuring that each veneer is optimized for its particular intended position within the set of teeth.

In an embodiment of a dental product according to the invention, the ceramic material is translucent. This provides for the dentist or any other skilled person applying the veneer onto the selected tooth the possibility to improve the appearance in terms of the color of the tooth by adding a suitable color agent to the bonding material for attaching the tooth veneer to the tooth. This enables obtaining an optimal color of the tooth at the final stage of the procedure. The involvement of a dental technician for obtaining a preferred color of the veneer is not necessary, keeping down costs and time for the person involved.

In an embodiment of a dental product according to the invention, the product comprises a plurality of tooth veneers for one set of teeth. Often the plurality of tooth veneers will be suitable for covering the upper six frontal teeth. Advantageously, the application of the tooth veneer(s) can take place in one session, so that undesired differences in the color of the individual teeth can be avoided. It will also be possible, if so desired, to achieve a natural grading of the color along the set of teeth so that the tooth veneer applied next to a tooth which is not covered by a tooth veneer will not show a sharp contrast to the color of the uncovered tooth.

In an embodiment of a dental product according to the invention, the at least one tooth veneer comprises a pre-treated bonding material, such as at least partially cured bonding material cured before application to a tooth. It is then further possible that bonding material comprises at least one coloring material and the at least one tooth veneer is at least partially transparent or translucent The invention also provides a method of improving the aesthetics of a tooth, wherein the method comprises placing a dental product as described above onto a tooth surface.

### BRIEF DESCRIPTION OF THE DRAWINGS

The invention will further be explained with reference to the drawing, in which:
Fig 1 shows a set of teeth and an embodiment of a veneer in accordance with the invention;
Fig. 2 shows a single tooth and an embodiment of a veneer in accordance with the invention,
Fig. 3 shows the tooth and veneer shown in Fig. 2, wherein the veneer is placed onto the tooth;
Fig. 4 shows a three dimensional representation of a tooth and steps for making an embodiment of a dental product in accordance with the invention;
Fig. 5 shows schematically a three dimensional representation of an intermediate product in a production process of an embodiment of a dental product in accordance with the invention;
Fig. 6 shows a support to be used in a production process for producing an embodiment of a dental product in accordance with the invention;
Fig. 7 shows an intermediate result of a production process for producing an embodiment of a dental product in accordance with the invention;
Fig. 8 shows an intermediate result of a production process for producing an embodiment of a dental product in accordance with the invention;
Fig. 9 shows an assembly for temporarily holding an embodiment of a dental product in accordance with the invention.
Fig. 10 shows a first part of an assembly for temporarily holding an embodiment of a dental product in accordance with the invention;
Fig. 11 shows use of an assembly for temporarily holding an embodiment of a dental product in accordance with the invention;
Fig. 12 shows schematically and in cross section an assembly for temporarily holding an embodiment of a dental product in accordance with the invention; and
Fig. 13 shows schematically a top view of the assembly shown in Fig. 12 without its container lid, as viewed in the direction of arrow A1.

### DESCRIPTON OF EMBODIMENTS

Specific embodiments of the invention will now be described with reference to the accompanying drawing. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawing is not intended to be limiting of the invention. In the drawing, like numbers refer to like elements.

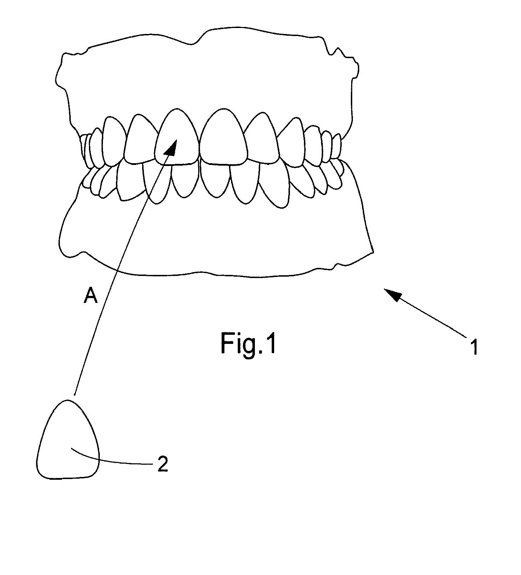

Fig. 1 shows a set of teeth 1 as well as a dental product 2, in this example being one tooth veneer 2. Such a veneer may also be referred to as a laminate. In the following, reference will be made only to a veneer, which should not be construed as limiting. A laminate is also embraced by the term veneer. Arrow A shows where the tooth veneer 2 is intended to be placed within the set of teeth 1. The veneer 2 is ready for application in the sense that no further re-shaping of the veneer 2 has to take place. The veneer 2 concerns an individualized dental product. The veneer 2 has characteristics which are related to the characteristics of a tooth of a person. One side (the back surface) of the veneer is designed to match the tooth 3 it is meant to cover. The veneer 2 bears, as such, features which correspond to features of an individual, namely the person for whom the veneer 2 is designed. Given that match, there will not be a need to remove material from the tooth 3.

For certain embodiments of the invention, it is described that it is not necessary to remove material from the tooth. In other embodiments, small portions of a tooth may be prepared, e.g. by grinding. This may be done e.g. to remove imperfections of a tooth surface. However, it is not intended that an entire surface, such as a front surface of a tooth, is prepared by grinding, e.g. since the veneer is sufficiently thin to not cause discomfort for the person wearing the veneer 2.

The veneer 2 may be individually designed to correspond to at least a part of the tooth 3 which is meant to be covered by the veneer 2. The tooth 3 as covered with the veneer 2 will increase its thickness by the layer of veneer 2, and by a very thin layer of bonding material 4 which is placed between the veneer 2 and the tooth 3 for bonding the veneer 2 to the tooth 3.

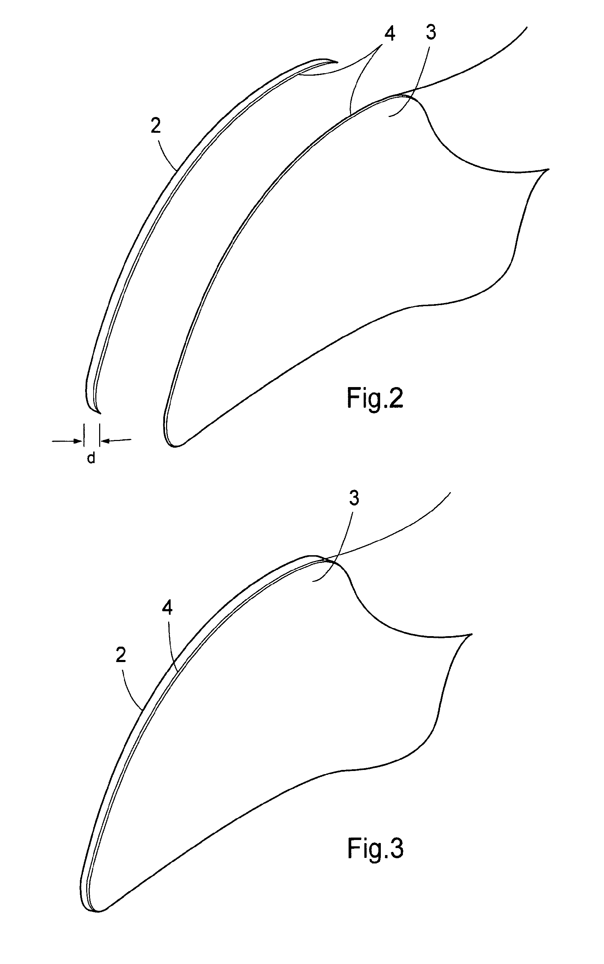

Fig. 2 shows a cross-sectional view of a tooth 3 which is to be covered by the veneer 2. For applying the veneer 2 to the tooth 3, surfaces of the veneer 2 and the tooth 3 facing each other in that application may each be etched in order to provide clean surfaces. This is normal standard practice, routinely carried out by a dental practitioner. The etching may be followed by applying a silanization layer to each of the surfaces. Also this concerns a standard practice, routinely carried out by a dental practitioner. The silanization layer provides a sealing to the etched surface. After this, a thin layer of bonding material 4 can be applied to each of these surfaces, i.e. to the veneer 2 and the tooth 3. This bonding material 4 can be provided with a color agent (not shown). The veneer 2 is then applied to the tooth 3 to end up with a tooth 3 covered by veneer 2 and bonding material 4, as shown in Fig. 3. It is to be noted that, effectively, no tooth material has been removed from tooth 3. Hence, the veneer 2 is applied to a so-called "non-prepared" tooth. The veneer 2 is individually designed to correspond to a part of a non-prepared tooth 3 that is covered by the veneer 2. The bonding may be further facilitated by light-curing, as is well known in the art.

In some embodiments, the tooth veneer 2 is pre-treated with a bonding material before application onto the person's tooth and possibly even before delivery to the dental practitioner applying the tooth veneer 4. Using conventional veneers, the bonding material is typically applied to the tooth 3 onto which the veneer is to be applied, the veneer is then placed on that tooth, and the bonding material cured, such as by light curing. According to embodiments of the invention, the bonding material is at least partly applied to the tooth veneer 2, such as 25-75% of the total bonding material to be used for bonding the tooth veneer 2. The pre-treatment can be carried out at the production facility of the tooth veneer 2 or by a dental technician. The pre-treatment of at least one side of the tooth veneer, such as the backside facing the tooth 3, may comprise at least one of the following steps: cleaning of the tooth veneer; application of etching gel for preparing a chemical and/or mechanical connection surface; providing etched surface prepared for sealing with silane as transport protection; applying a silanization process for obtaining a silanized surface; application of at least one layer of bonding material 4 on top of the silanized surface; application of coloring material, such as for cervical and/or incisal colorings, before, on top of, or mixed in the bonding material; applying the bonding material to the tooth veneer 2; and light curing the bonding material 4 for delivery to the dental practitioner.

Embodiments may also comprise pre-treatment by the practitioner of the tooth to receive the tooth veneer 2. The pre-treatment by the practitioner may comprise at least one of the following steps: cleaning the non-prepared (i.e. substantially non-ground front surface) surface of the tooth 3, such as with isopropanol; etching of the tooth surface, such as with etching gel, e.g. fluoride etching gel, fluoride hydrogen etching gel etc.; silanization of the etched tooth surface; and application of the remainder of the bonding material 4, such as 25-75 % if 25-75% was applied to the tooth veneer, onto the tooth surface to end up with a total of 100% of required bonding material. The bonding material applied to the tooth surface may have a neutral color, such as transparent or white, whereby appearance provided by the coloring added to the veneer 2 is substantially not affected.

Embodiments of the invention may also comprise at least one of the following steps for a veneering procedure: providing pre-treated veneer 2 with hardened bonding material; applying the tooth veneer onto the tooth surface comprising bonding material 4; adjusting the veneer 2; and curing, such as by light curing, the connection between the surfaces with bonding material 4.

Using embodiments of the pre-treating procedure, the veneer 2 may comprise a pre-treated bonding material. The pre-treated bonding material 4 may comprise an at least partially cured bonding material 4, such as a light curable dental bonding agent. The pre-treated bonding material may comprise at least one coloring material to color the veneer 2. Hence, due to the thinness of the veneer 2 in combination with its translucency the coloring of the bonding material 4 will be visible. Therefore, application of a separate coloring layer is not necessary.

As can easily be seen in Figs. 2 and 3, the veneer 2 is plate-like. The veneer 2 has a plate thickness "d" of at least about 0.08 mm. The thickness "d" will be less than 0.2 mm. It is more preferable that the thickness "d" of the veneer is less than 0.14 mm. Ideally, the plate thickness "d" is less than 0.1 mm. The plate thickness "d" is a mean thickness. At various portions the veneer 2 may be thinner or thicker. For example, the veneer 2 may comprise various structures to look more natural, such as grooves or furrows. Additionally or alternatively, the tooth veneer may comprise a texture at the front surface for a more natural look of the veneer 2 or to recreate a lost feature of a person's dentition, e.g. due to abrasion. It is also thinner at the border of the veneer 2. It has advantegously shown that the preferred ranges of the thickness is sufficiently thin to not cause discomfort to the person wearing the veneer 2 without removing tooth material and yet provide sufficient strength of the tooth veneer.

Furthermore, in embodiments of the invention, the veneer 2 or laminate is a non-framework dental product, i.e. the veneer or laminate is placed directly on to a tooth surface with out any intermediate framework to strengthen the laminate or veneer. Other dental prosthesis, such as a dental crown or bridge, or even some ceramic veneers, may include a veneering layer on top of a framework, such as a coping or bridge framework, wherein the veneering layer is applied in several layers on top of the framework or coping and subsequently sintered. However, such a veneer 2 on top of a framework does not suffer from strength issues and is not particularly fragile to handle.

The veneer 2 is of a ceramic material, which is preferably translucent. Obtaining a desired color of a tooth 3 can more easily be obtained at the "placement site" by adding a color agent to the bonding material 4 and then applying the translucent ceramic veneer 2, so that the color agent can contribute to the appearance of tooth 3, in terms of its color.

The ceramic material may be a glass ceramic material ,such as a Li-disilicate glass ceramic. Li-disilicate glass ceramic has a high strength, which provides for improved possibilities to handle the fragile product. Such glass-ceramic is e.g. available under the tradename IPS e.max from Ivoclar Vivadent, Lichtenstein. Alternatively, the ceramic material may comprise a microwave sintered aluminium oxide ceramic. Such materials are particularly useful in combination with a veneer having a thickness within the ranges of embodiments of the invention.

In some embodiments, the veneer 2 comprises a single layer of glass-ceramic having uniform composition. Optionally, non-ceramic coloring and/or glazing layers may be added at least on one side of the veneer 2.

An individualized dental product 2ready for application, such as a ceramic veneer 2, can be produced as follows.

For designing the veneer 2, use can be made of computer-readable tooth data which represent a predetermined tooth for which the veneer 2 is designed. These data can be obtained by a standard method, using standard systems, well known in the art and widely available. For example, such methods may comprise scanning of an impression or a cast model based on the impression to obtain the computer-readable data. On the basis of the computer-readable tooth data it is possible to produce on a display a three-dimensional representation 22 of the predetermined tooth 3 for which the veneer 2 is designed. Standard tools within an dental CAD program enable visualizing the three dimensional representation 22 as seen from a different angle, after rotation, as zoomed in, as zoomed out, as fully transparent etc. Although ideally the complete predetermined tooth 3 is shown, it is possible that only a part of the predetermined tooth 3 is shown.

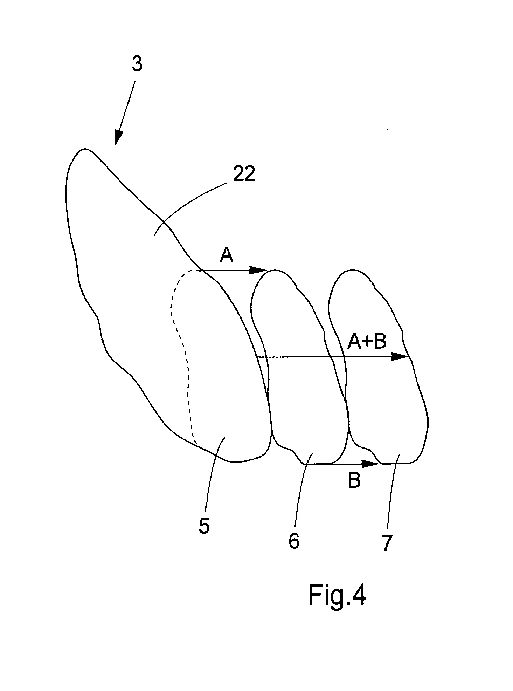

On the basis of the three-dimensional representation 22, computer-readable veneer data are provided for determining the geometry of the veneer. Fig. 4 shows schematically how this can be done. In the three-dimensional representation 22 a selected tooth part 5 is defined. The selected tooth part 5 is to be covered by the veneer 2. The selected tooth part 5 can, for instance, be defined by providing with the cursor a line, such as a finish line, in the three-dimensional representation of the predetermined tooth 3 for which the veneer 2 is designed. In a standard way, it can be confirmed to the computer that the line, closed in itself, defines indeed the selected tooth part 5. On the basis of the selected tooth part 5, a back surface 6 of the veneer 2 is defined. On the basis of the selected tooth part 5, a front surface 7 of the veneer 2 is defined. Defining the back surface may comprise copying the selected tooth part 5. Defining the front surface 7 may also comprise copying (directly or indirectly) the selected tooth part 5. Defining the back surface 6 of the veneer 2 can be based on a reproduction of the selected tooth part visualized in a three-dimensional representation 22, possibly with an offset (A), e.g. to provide space for the bonding material 4. Likewise, defining the front surface 7 of the veneer 2 can be based on a reproduction of the selected tooth part 5, or a reproduction of the back surface 6, visualized in the three-dimensional representation 22 with an offset (A+B, B), wherein B represents the thickness of the veneer 2, relative to the selected tooth part 5 and/or to the back surface 6. Having defined the back surface 6 and the front surface 7, it is possible to define according to a predetermined program a connection between edges of the back surface 6 and edges of the front surface 7. In any case, by this method it is possible to obtain computer-readable veneer data, defining the geometry of the veneer 2. It may further be possible to define in addition to the veneer data computer-readable sprue data for one or several sprues.

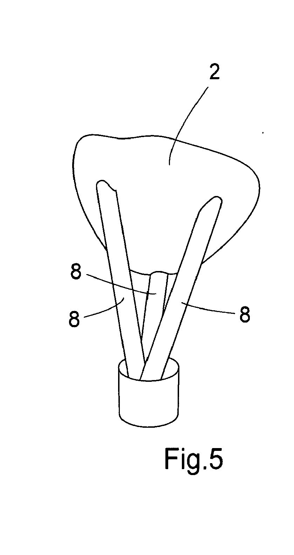

Fig. 5 shows a veneer 2 and three sprues 8 usable (or used) for producing a veneer 2 by casting or an injection molding method. It is of course possible that only one sprue 8 or two sprues 8 are used for producing the veneer 2. Having defined the veneer data and possible sprue data, it is possible to provide in three dimensions a master 10 of the veneer with any designed sprue. One example of a CAM (Computer Aided Manufacturing), the technology that can be employed to provide the master 10, is often referred to as "rapid prototyping", which may comprise stereolithography, selective laser sintering, or inkjet printing. Alternatively, milling is employed as the CAM technology for providing the master 10. Such three-dimensional production methods are well known in the art, such as from Solidscape, USA, 3D System, USA, Objet, Israel, or Envisiontech, Germany.

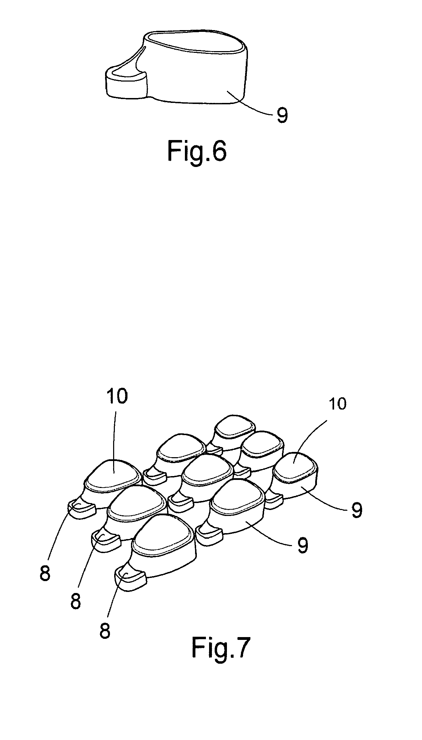

Fig. 6 shows a print support 9 onto which a shape of the individualized tooth veneer or the master 10 to be produced and any sprue 8 can be provided. The print support may have a surface corresponding to a surface of the master 10 and thus to the veneer 2. The print support 9 may be individually produced based on the planned data for the tooth veneer and by any of the techniques discussed above for producing the master 10. Fig. 7 shows a result of such a production process. An individualized tooth veneer 2 and one sprue 8, connected thereto, rest on the top of each print support 9.

The veneer 2 can also be provided by milling, such as according to any of the methods described in WO2005/046502, which is incorporated herein by reference for any purpose, for providing a dental prosthesis.

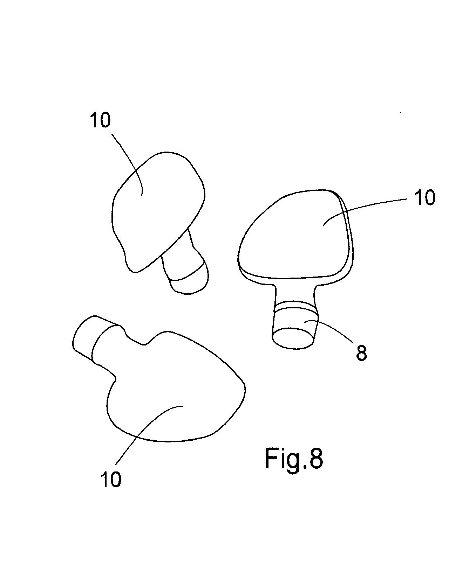

Fig. 8 shows the shape of the master 10 of the individualized tooth veneer 2 and the sprue 8, as removed from the print support 9.

The master 10 may be used to cast or mold the veneer 2. In short, the master 10 of the individualized veneer, as optionally provided with one or more sprues 8, commonly also referred to as "the mold", can be placed in a so-called *cuvette*. The mold 10, as placed in a *cuvette* is surrounded by an investment material. In this method the mold, i.e. the master 10 with any sprues is burned out from the *cuvette* so that a cavity is formed for forming the veneer 2, and the one or more sprues 8, by the ceramic material of which the veneer 2is to be made. The material is supplied to this cavity and pressed into the space under vacuum and at high temperature. These steps are all well known in the art and the process or sintering parameters, such as pressure, temperature and time depend on the material used. Once a veneer 2 and sprue 8 have been formed in the desired shape, demolding takes place, for instance by blasting the investment material. The sprue 8 can be removed by standard methods, such as polishing or milling.

The produced tooth veneer 2 can be pre-treated, such as discussed above, before it is transported.

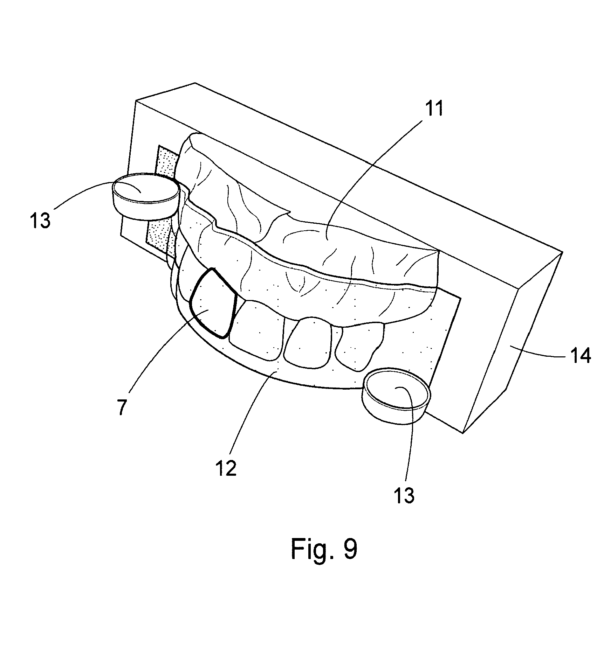

Once the veneer 2 has been produced, it needs to be temporarily held, possibly transported, and finally be applied to the tooth 3 for which the veneer 2 has been designed. For that purpose, the dental product 2 is associated with a first and a second part 11, 12 for sandwiching each of the tooth veneers 2 thereinbetween. Fig. 9 shows an example of such first and second parts 11,12. The first part 11 is provided with a shape for fittingly matching one side of the plate-like tooth veneer. As shown in the example of Fig. 9, the first part may comprise a model of a set of teeth. The second part 12 is arranged for releasably bonding another side of the plate-like tooth veneer 2 against that second part 12. The second part 12 comprises a foil, which may also be a tape. As shown in Fig. 9, when the first and second part are sandwiching the tooth veneer 2, the first and second part 11, 12 are releasably securable to each other, in this example by sticking the tape against the model. The second part 12 may be provided with grasp holders 13 for manually holding the second part 12, for instance during placement of the veneer 2 against the predetermined tooth 3.

The releasable bonding may be provided by, .e.g. vacuum bonding, electrostatic bonding, and/or an adhesive bonding film. Vacuum bonding can be provided by a thermo-forming unit, wherein a deformable blank is pre-heated, positioned on top of the first part with the veneer 4 inbetween and deformed by activation of a vacuum pump, whereby the blank releasably bonds to the veneer 2, which is supported by the first part 11. Then, the deformed blank can be trimmed to a desired shape. Such vacuum forming apparatus is available from, e.g., Dreve, Germany, under the tradenames Vacformat U, Vacformat 2000 and Druformat Scan. Bonding films, such as electrostatic foils or films, films with adhesives, both resin based and light curing based, etc. are available, from, e.g., 3M, USA.

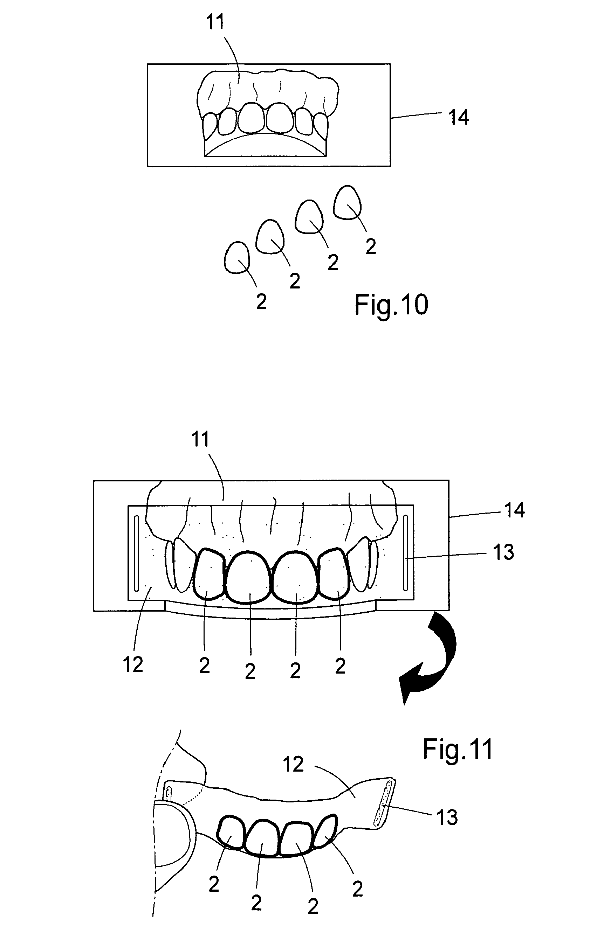

As shown in Fig. 10, the dental product may comprise a number of individualized veneers 2, all for one set of teeth. As shown in Fig. 11, it will be possible that on separating the second part 12 from the first part 11, each of the tooth veneers as bonded to the second part 12 is manually transferred to the front teeth to which the veneers 2 have to be applied. It is then possible to attach each of the veneers 2 individually, by applying bonding material 4 only to one predetermined tooth or corresponding tooth veneer 2 at a time, or applying bonding material 4 to each of the tooth veneers 2 simultaneously, therewith reducing "chair time" and improving the probability that the veneer 2 is applied without disturbing the relative position to one another.

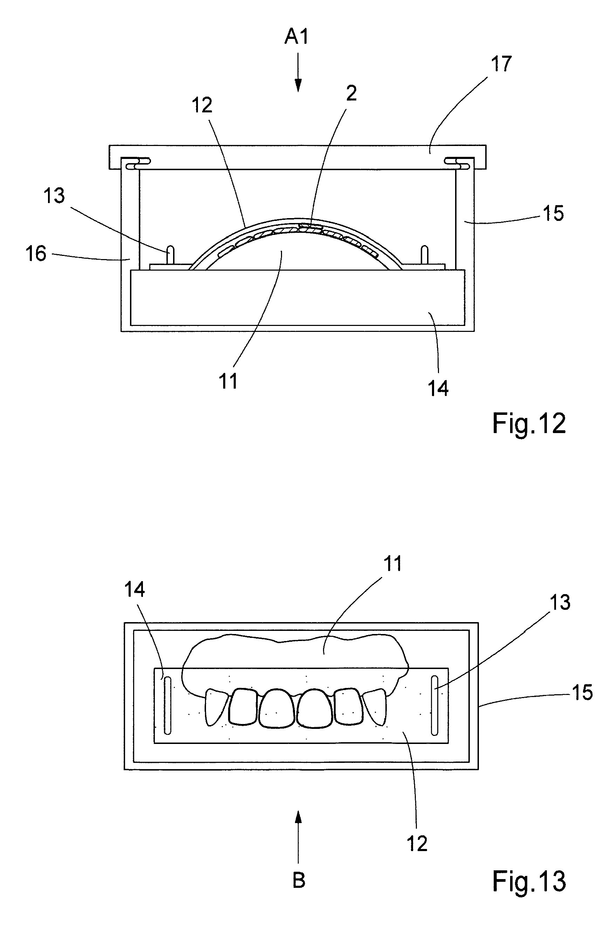

Figures 12 and 13 show schematically two cross sections of an embodiment wherein the model is associated with the support 14 for supporting the model. Fig. 13 shows a top view of the embodiment shown in Fig. 12 (without a container lid) as viewed in the direction of arrow A in Fig. 12. Fig. 12 shows a cross section viewed in the direction of arrow B in Fig. 13. Support 14 and the model are positionally fixed relative to each other. In the embodiment shown, the first part 11 concerns a single piece part, i.e. the support 14 and the model are integrally connected to each other. However, it is also possible that the support 14 and the model are two separate parts which are fixed or fixable to each other. The assembly schematically shown in Figs. 12 and 13 further comprises a container 15. The support 14 has dimensions for holding the model stable in the container 15. The container 15 may for that purpose for instance internally be provided with a shoulder 16 under which edges of the support 14 can be clamped. The material of the container 15 may be of a thermoplast which allows for slight bending of parts of the container. This would allow for placing the support 14 as shown in Fig. 12 and for closing the container 14 with a container lid 17 by means of a clamping mechanism or snap-fit, or any other mechanism known in the art for providing a closed container 15.

A dental product 2 as described above, comprising one or more tooth veneers 2, on application onto predetermined teeth 3, improves the aesthetics of these teeth 3. Although it is very possible that the application can occur as described above, and with the use of the second part 12, the foil with grasp holders 13, as a handling and placement tool, it is also possible that a dental practitioner rather makes use of tools having suction cups and/or vacuum tweezers for handling such a thin veneer. A method of improving the aesthetics of the tooth, may comprise placing a dental product 2 as described above, and may further comprise the use of a bonding material 4 to which a color agent has been added.

The invention is not limited to the embodiments discussed above. Many modifications and different embodiments are possible. Each of these are understood to fall within the framework of the invention, as defined by the appended claims.

## Claims

**1.** An individualized dental product (2) ready for application comprising:
at least one tooth veneer which is of a ceramic material, is plate-like and has a plate thickness of at least about 0.08 mm, wherein the thickness is less than 0.2 mm, less than 0.14 mm or less than 0.1 mm.

**2.** A dental product (2) according to claim 1, wherein each of the at least one tooth veneer (2) is individually designed to match a part of a predetermined tooth (3)that is to be covered by that veneer (2).

**3.** A dental product (2) according to claim 1 or 2, wherein each of the at least one veneer (2) is individually designed to correspond to a part of a non-prepared tooth that is to be covered by that veneer (2).

**5.** A dental product (2) according to any one of the preceding claims, wherein the ceramic material is translucent.

**6.** A dental product (2) according to any one of the preceding claims, wherein the product comprises a plurality of tooth veneers.

**7.** A dental product according to any one of the preceding claims, associated with a first and a second part (11, 12) for sandwiching one of the at least one tooth veneer (2) thereinbetween, wherein the first part (11) is provided with a shape for fittingly matching one side of one of the at least one plate-like tooth veneer (2) and the second part (12) is arranged for releasably bonding another side of the at least one plate-like tooth veneer (2) against the second part (12) and optionally comprises at least one grasp holder (13).

**8.** A dental product (2) according to claim 7, wherein the first part (11) and the second part (12) are releasably securable to each other when the first part (11) and second part (12) are sandwiching the at least one tooth veneer (2).

**9.** A dental product (2) according to claim 7 or 8, wherein the first part (11) comprises a model of at least a part of a person's set of teeth.

**10.** A dental product (2) according to any of the preceding claims, wherein the ceramic material is glass ceramic material, such as a Li-disilicate glass ceramic, or a microwave sintered aluminium oxide ceramic.

**11.** A dental product (2) according to any one of claims 7-9, wherein the second part (12) comprises a foil.

**12.** A dental product (2) according to any one of claims 9-11, wherein the model is associated with a support (14) for supporting the model, the support (14) and the model being positionally fixed or fixable relative to each other and optionally integrally connected, wherein the support (14) is associated with a container (15) and wherein the support (14) has dimensions for holding the model stable in the container (15).

**13.** A dental product (2) according to any one of the preceding claims, wherein the at least one tooth veneer (2) comprises a pre-treated bonding material (4), such as an at least partially cured bonding material cured before application to a tooth (3).

**14.** A dental product (2) according to claim 13, wherein the bonding material (4) comprises at least one coloring material and the at least one tooth veneer (2) is at least partially transparent or translucent.

**15.** A method of improving the aesthetics of a tooth (3), wherein the method comprises placing a dental product (2) as defined in any one of claims 1-6, 10, or 13-14 onto a tooth surface.
